# EUROPÄISCHE PATENTSCHRIFT

(11) **EP 0 694 515 B1**
(45) Veröffentlichungstag und Bekanntmachung des Hinweises auf die Patenterteilung: **18.08.1999**
(21) Anmeldenummer: 95106360.1
(22) Anmeldetag: 27.04.1995
(51) Int. Cl.: C07C 31/26, C07C 29/141, C07H 3/02, C07H 3/04, C07H 15/04, B01J 23/85, B01J 23/86, B01J 23/88

(54) **Verfahren zur Hydrierung von Zuckern**
Process for hydrogenation of sugars
Procédé pour l'hydrogénation des sucres

(30) Priorität: 10.05.1994 DE 4416408
(43) Veröffentlichungstag der Anmeldung: 31.01.1996
(73) Patentinhaber: BAYER AG, 51368 Leverkusen (DE)
(72) Erfinder: Darsow, Gerhard, Dr., D-47804 Krefeld (DE)

(56) Entgegenhaltungen:
- EP-A- 0 423 525
- US-A- 3 691 100
- DATABASE WPI Week 9241 Derwent Publications Ltd., London, GB; AN 92-334368 XP002006489 & HU-A-60 230 (MTA KOEZPONTI KEMIAI KI) , 28.August 1992
- DATABASE WPI Week 7602 Derwent Publications Ltd., London, GB; AN 76-033375 XP002006490 & SU-A-453 187 (N-CAUCAS BIO-SYNTH) , 30.April 1975 & STN-INFORMATION-SERVICE FILE CA,

## Beschreibung

Die Erfindung betrifft ein kostengünstiges Verfahren zur kontinuierlichen katalytischen Hydrierung von Zuckern, wie z.B. D-Xylose, α-D-Glucose, 4-O-β-D-Galactopyranosyl-α-D-glucopyranose oder 4-O-α-D-Glucopyranosyl-α-D-glucopyranose, mit Wasserstoff zu den entsprechenden Zuckeralkoholen, wie z.B. Xylit, Sorbit, 4-O-β-D-Galactopyranosyl-D-sorbit oder 4-O-α-D-Glucopyranosyl-D-sorbit.

Der Reaktionsverlauf läßt sich durch folgende Reaktionsschemen veranschaulichen:

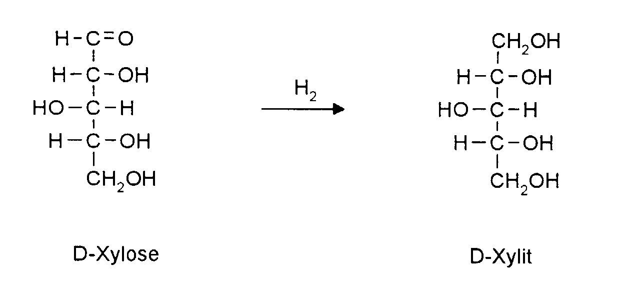

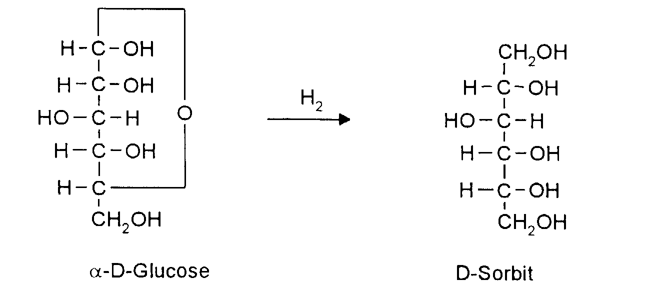

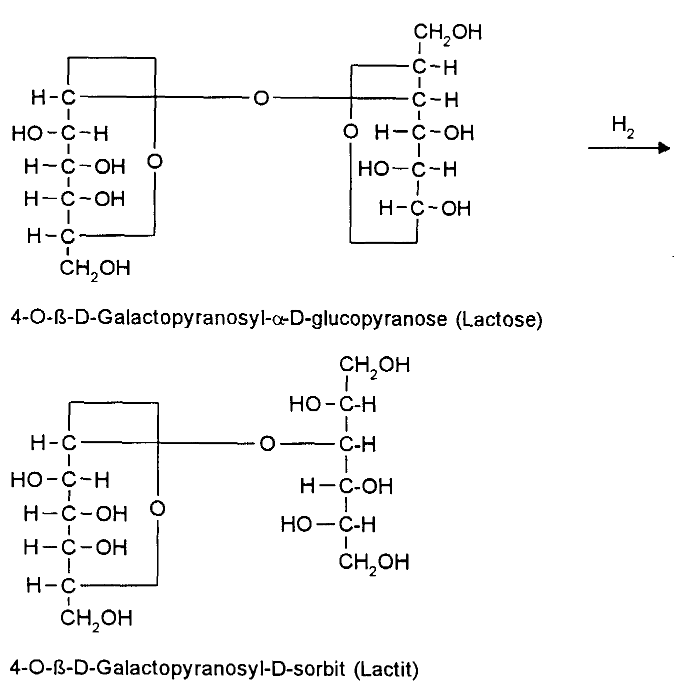

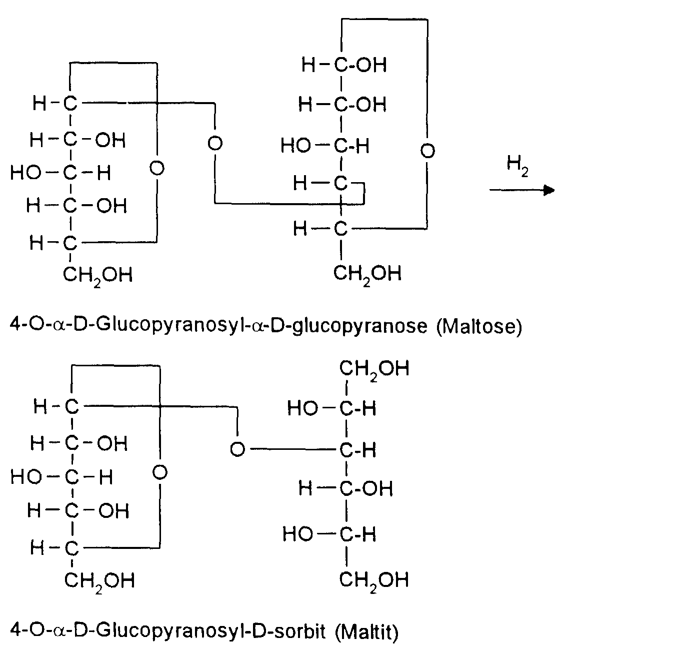

Zur Herstellung von Xylit oder Sorbit wird meistens ein diskontinuierliches Verfahren angewandt, bei dem ein pulverförmiger Nickelkatalysator in einem Suspensionsverfahren zum Einsatz kommt.

Auch zur Herstellung des bisher in der Natur nicht nachgewiesenen Lactits ist aus EP-A 39 981 ein diskontinuierliches Verfahren bekannt, bei dem ein pulverförmiger Nickelkatalysator in einem Suspensionsverfahren zum Einsatz kommt.

Ein solches Verfahren wird auch in der US-PS 3 741 776 zur Herstellung des Maltits vorgeschlagen.

Diskontinuierliche Verfahren haben den Nachteil, daß ihre Kapazität, relativ zum Reaktionsvolumen, sehr klein ist und somit ein Bedarf nach großen Reaktionsapparaturen und Lagertanks besteht. Der Energieverbrauch ist unökonomisch, und der Personalbedarf ist verhältnismäßig hoch.

Kontinuierliche Pulverkatalysator-Verfahren, die mit mehreren in Kaskade geschalteten Hydrierreaktoren arbeiten, vermeiden einen Teil der genannten Nachteile. Es bleibt jedoch der umständliche Weg, den pulverförmigen Katalysator gezielt zu aktivieren, umzupumpen und quantitativ vom Reaktionsprodukt abzufiltrieren. Die Katalysatorschlammpumpen unterliegen einer hohen mechanischen Beanspruchung. Die quantitative Entfernung des pulverförmigen Katalysators ist aufwendig (Grob- und Feinfiltrationsapparate in Wechselausführung). Ferner ist die Gefahr groß, daß der Katalysator durch die zusätzlichen Operationen verhältnismäßig schnell seine Aktivität verliert (hoher Katalysatorverbrauch). Es ist daher wünschenswert, die Reaktion über fest angeordneten Katalysatoren ablaufen zu lassen, welche eine hohe spezifische Aktivität besitzen sollen, die auch über einen längeren Zeitraum von 1 bis mehreren Jahren nicht nachlassen soll, weil häufige Katalysatorwechsel auch bei Festbettreaktionen aufwendig sind.

Auch bei fest angeordneten Katalysatoren ist es üblich, mehrere Reaktoren hintereinander zu schalten, wodurch sich mehrere in Serie geschaltete Reaktionszonen ergeben (DE-OS 3 214 432).

Zum Einsatz kommen bisher überwiegend Nickelkatalysatoren auf oxidischem Trägermaterial (SiO₂/Al₂O₃) mit extrem hohen aktiven Oberflächen von 140 bis 180 m²/g, so daß die Katalysatoren derart aktiv sind, daß sie durch zusätzliche chemische Behandlungsmethoden stabilisiert werden müssen, beispielsweise durch Sauerstoffbegasung zur Bildung monomolekularer Sauerstoffschichten auf der Katalysatoroberfläche (DE-OS 3 110 493). Die desaktivierende Stabilisierung des Katalysators macht aber dann so hohe Reaktionstemperaturen bei der Hydrierung erforderlich (130 bis 180°C), daß unkontrollierbare Nebenreaktionen möglich werden, wie Verfärbung durch Karamelisierung und hydrierende Crackung (Hydrogenolyse) der Zuckeralkohole bis zur Bildung von Methanol und sogar Methan. Außerdem gehen bei dieser Reaktionsweise stets größere Anteile von Schwermetallen in ionischer oder kolloidaler Form in Lösung, was einmal eine nachträgliche Aktivkohlebehandlung des hydrierten Produktes und zum anderen eine Entionisierung durch Ionenaustauscher erforderlich macht.

Da die bekannten Hydrierverfahren mit auf pH-Werte von 7 bis 13 eingestellten Zuckerlösungen arbeiten, sind den Ausgangslösungen Alkalien oder Erdalkalien beizugeben, die ebenfalls vom Endprodukt wieder umständlich entfernt werden müssen (DE-OS 3 110 493; DE-OS 3 214 432).

Weiterhin ist zu erwarten, daß unter den hydrierenden Bedingungen eine merkliche Epimerisierung eintritt, so daß beispielsweise aus der D-Xylose neben Xylit auch Lyxit (bzw. Arabinit und Ribit) erhalten werden; aus α-D-Glucose wäre neben Sorbit auch Mannit zu erwarten.

Ferner ist der Effekt der Spaltung der Kohlenstoffkette von Zuckern bei der katalytischen Hydrierung von Raney-Nickel bekannt; DE-OS 2 756 270 beschreibt den Effekt an einem Zuckergemisch, wie es aus der Selbstkondensation von Formaldehyd hervorgeht, wobei im Rahmen der dortigen Ausführungsbeispiele eine deutliche Verschiebung von höheren C-Kettenzahlen zu niedrigeren beobachtet wird.

Aus der EP-A 423 525 ist ein Verfahren zur kontinuierlichen Hydrierung von Zuckern zu den entsprechenden epimerenfreien Zuckeralkoholen über trägerfreien Festkörpern von Elementen der 8. Nebengruppe des Periodensystems bekannt, wobei diese trägerfreien Formkörper vorzugsweise durch Verpressen und/oder Verkleben von Metallpulver hergestellt worden sind. Hierbei ergab sich, daß die Zucker nicht nur nahezu vollständig umgesetzt werden, sondern unter Vermeidung von Epimerisierung und C-Kettenspaltung sowie unter Vermeidung der Bildung höhermolekularer Komponenten durch Kondensationsreaktionen unter Etherbildung nur jeweils ein Zuckeralkohol erhalten wird. Es wäre allerdings wünschenswert, die hohen Katalysatorkosten zu senken. Außerdem ist man immer bestrebt, ein Verfahren bei möglichst niedriger Temperatur durchzuführen, um die Energiekosten zu senken.

Es wurde jetzt überraschenderweise gefunden, daß Elemente der VI. Nebengruppe des Periodensystems enthaltende Metallpulver von Nickel, Kobalt und Eisen oder ihrer Legierungen nach ihrer Verpressung zu Formkörpern nicht nur ebensogut die Hydrierung der genannten Zucker zu epimerenfreien Zuckeralkoholen katalysieren, sondern daß Katalysatoren aus diesen Metallen bzw. Metall-Legierungen, die um 30 bis 45 % preiswerter sind, sogar eine erheblich höhere Hydrieraktivität aufweisen, so daß die Hydrierreaktion bei einer bis zu 30°C niedrigeren Reaktionstemperatur durchgeführt werden kann. Dabei können die zum Einsatz kommenden Pulver zusätzlich gewisse Anteile (max. zulässig 10 Gew.-%) anderer nicht katalytisch wirkender Metalle (z.B. Mangan, Silicium, Aluminium, Titan) enthalten, ohne daß die hohe Aktivität gemindert wird.

Gegenstand der Erfindung ist daher ein Verfahren zur Herstellung von Zuckeralkoholen aus der Gruppe von Xylit, Sorbit, 4-O-β-D-Galactopyranosyl-D-sorbit (Lactit) und 4-O-α-D-Glucopyranosyl-D-sorbit (Maltit) durch katalytische Hydrierung der korrespondierenden Zucker D-Xylose, α-D-Glucose, 4-O-β-D-Galactopyranosyl-α-D-glucopyranose bzw. 4-O-α-D-Glucopyranosyl-α-D-glucopyranose in wäßriger Lösung mit Wasserstoff unter erhöhtem Druck und bei erhöhter Temperatur, dadurch gekennzeichnet, daß man die Hydrierung kontinuierlich bei einem Wasserstoffdruck von 100 bis 400 bar, vorzugsweise 150 bis 300 bar und Temperaturen von 40 bis 80°C, vorzugsweise 55 bis 70°C, im Festbettverfahren in einer Reaktionszone über als Hydrierungskatalysatoren dienenden trägerfreien Formkörpern mit einer Druckfestigkeit von 20 bis 250 N, vorzugsweise 110 bis 220 N, und einer inneren Oberfläche von 10 bis 80 m²/g von (i) einem oder mehreren Elementen der Eisenuntergruppe der VIII. Nebengruppe durchführt, die zusätzlich mit (ii) aktivierend wirkenden Elementen der VI. Nebengruppe legiert sind.

Die Druckfestigkeit der trägerfreien Formkörper kann nach DIN 50 106 bestimmt werden.

Die Überprüfung von trägerfreien Formkörpern auf die anspruchsgemäßen inneren Oberflächen und damit auf Brauchbarkeit für das erfindungsgemäße Verfahren können nach Methoden durchgeführt werden, die von F.M. Nelsen und F.T. Eggertsen, Analyt. Chem. 30 (1958), 1387 bzw. S.J. Gregg und S.W. Sing, Adsorption, Surface Area and Porosity, London 1967, Kap. 2 und 8, beschrieben worden sind.

Die Eisenuntergruppe der VII. Nebengruppe des Periodensystems enthält die Elemente Eisen, Cobalt und Nickel. Die erfindungsgemäß zu verwendenden trägerfreien Formkörper enthalten eines oder mehrere dieser Metalle in Mengen von mindestens 60, vorzugsweise mindestens 70, insbesondere mindestens 80 Gew.-%, bezogen auf trägerfreie Formkörper.

Die VI. Nebengruppe des Periodensystems enthält die Elemente Chrom, Molybdän und Wolfram. Die erfindungsgemäß zu verwendenden trägerfreien Formkörper enthalten eines oder mehrere dieser Metalle in Mengen von mindestens 0,1, vorzugsweise mindestens 0,3, insbesondere mindestens 0,5 Gew.-%, bezogen auf trägerfreie Formkörper; sie enthalten eines oder mehrere dieser Metalle in Mengen von höchstens 20, vorzugsweise höchstens 10 und insbesondere höchstens 5 Gew.-%, bezogen auf trägerfreie Formkörper.

Die erfindungsgemäß zu verwendenden trägerfreien Formkörper können darüber hinaus - jeweils bezogen auf trägerfreien Formkörper - bis zu 20, vorzugsweise bis zu 15, insbesondere bis zu 10 Gew.-% anderer Metalle enthalten; Beispiele solcher Metalle, die nicht katalytisch wirken müssen, umfassen Aluminium, Silicium, Titan und Mangan. Nach einer bevorzugten Ausführungsform enthalten die trägerfreien Formkörper außer den Komponenten (i) und (ii) nicht mehr als 10 Gew.-% Aluminium und nicht mehr als 5 Gew.-% anderer Metalle.

Das erfindungsgemäße Verfahren gestattet die Herstellung der Zuckeralkohole in einer Reinheit von über 99 % in der Trockenmasse. Der Gehalt an nicht umgesetztem Zucker erreicht Werte von 0,2 Gew.-% oder darunter. Da die glykosidischen Etherbindungen bei Disacchariden geschont werden, liegen die Gehalte der Monozuckeralkohole bei 0,3 Gew.-% oder darunter. Die handelsüblichen Spezifikationen für die Zuckeralkohole, etwa nach DAB, USP oder FCC, können daher ohne weitere Reinigungsvorgänge direkt erfüllt werden.

Als Ausgangsverbindung für das erfindungsgemäße Verfahren kann kristalline D-Xylose, α-D-Glucose, α-Lactose oder α-Lactose-Monohydrat bzw. Maltose (flüssig oder in ihrer β-Form auch als kristallines Monohydrat) eingesetzt werden. Der Einsatzstoff wird vorzugsweise in sauerstofffreiem entionisiertem Wasser so gelöst, daß eine 15 bis 45 gew.-%ige, bevorzugt 35 bis 40 gew.-%ige Lösung entsteht, deren pH-Wert 3,5 bis 10,5 beträgt. Die Lösung der Monosaccharide wird bevorzugt auf einen pH-Wert von 3,5 bis 8, die der Disaccharide bevorzugt auf einen pH-Wert von 5,5 bis 10,5 eingestellt. Für alle Ausgangsstoffe ist der besonders bevorzugte pH-Bereich 6 bis 7,5. Die als Ausgangsverbindungen genannten Zucker zeigen, gelöst in Wasser mit einem pH-Wert von 7, eine neutrale oder, bedingt durch spurenweise Bildung von Zuckersäuren, eine schwach saure Reaktion, können aber beispielsweise durch gezielte Zugabe von basisch reagierenden wasserlöslichen Verbindungen, wie Alkalicarbonaten oder bevorzugt Ammoniak in wäßriger Lösung, oder sauer reagierenden Verbindungen, wie Zuckersäuren, Sorbinsäure oder Zitronensäure, auf den gewünschten pH-Wert eingestellt werden.

Für das erfindungsgemäße Verfahren wird reiner Wasserstoff eingesetzt, der auf einen Druck von 100 bis 400 bar, bevorzugt 150 bis 300 bar, vorkomprimiert wird. Die Hydrierung erfolgt kontinuierlich im Festbettverfahren an den als Hydrierkatalysatoren dienenden trägerfreien Formkörpern, indem man die zu hydrierende Lösung entweder im Gleichstrom von unten aufsteigend gemeinsam mit dem vorher zugemischten Wasserstoff über den in einem Hydrierreaktor angebrachten Katalysator strömen läßt (Gleichstromverfahren) oder aber, indem man die von unten aufsteigende zu hydrierende Lösung dem von oben einströmenden Wasserstoff entgegenführt (Gegenstromverfahren).

Der Hydrierreaktor kann entweder ein einzelnes Hochdruckrohr aus Stahl oder einer Stahllegierung sein, das mit dem trägerfreien Formkörper ganz oder teilweise gefüllt wird, wobei bei gewissen Rohrquerschnitten auch die Anwendung des trägerfreien Formkörpers auf Horden (Drahtkörbe oder ähnliches) nützlich sein kann, oder aber ein ummanteltes Hochdruckrohrbündel, dessen Einzelrohre mit trägerfreien Formkörpern ganz oder teilweise gefüllt werden. Weiterhin kann anstelle eines größeren Einzelrohrreaktors eine Anordnung von mehreren kleinen Einzelreaktoren hintereinander in einer Kaskade betrieben werden.

Die Herstellung der trägerfreien Formkörper kann nach gebräuchlichen Methoden durch Verpressen der Metallpulver, beispielsweise auf Tablettier- oder Pelletiermaschinen, unter hohem Druck erfolgen, wobei zur Verbesserung des Haftvermögens der Metallpartikel auch Graphit und/oder Klebstoffe in Mengen von 0,5 bis 1 Gew.-%, bezogen auf das Gesamtgewicht der den Katalysator bildenden Bestandteile, zum Einsatz kommen können. Die Herstellung der trägerfreien Formkörper erfolgt vorzugsweise in einer sauerstofffreien Atmosphäre, um Oberflächenoxidation zu vermeiden. Beispiele für Formkörper sind Tabletten, Kugeln, Granulate mit Durchmessern von 3 bis 7 mm. Tablettierte Formkörper können weiterhin zur Vergrößerung der äußeren Oberfläche mit einer axialen Durchbohrung versehen sein. Solche Formkörper haben, makroskopisch betrachtet, eine glatte Oberfläche.

Die erfindungsgemäß einzusetzenden trägerfreien Formkörper besitzen Druckfestigkeiten von 20 bis 250 N, bevorzugt 110 bis 220 N. Dies ist wichtig, weil niedrigere Druckfestigkeiten zu einem Zerfall bzw. zu erosivem Abrieb der Formkörper führen, was eine unerwünschte Kontaminierung des Reaktionsproduktes mit Metallpulver bewirken würde.

Die Hydrierung wird bei Temperaturen von 40 bis 80°C, bevorzugt 55 bis 70°C durchgeführt. Niedrigere Temperaturen würden höhere Verweilzeiten oder den Verzicht auf einen weitgehend quantitativen Umsatz der Zucker ergeben. Höhere Temperaturen führen zu unkontrollierbaren Nebenreaktionen, wie Karamelisierung, Etherspaltung oder hydrierender Crackung, was zu Verfärbungen sowie Bildung unerwünschter Nebenprodukte führen kann.

Die stündliche Katalysatorbelastung kann bei 25 bis 100 g der als Ausgangsverbindungen benannten Zucker pro Liter Katalysator liegen. Bei Einhaltung der genannten Reaktionsbedingungen sind unerwartet hohe Katalysatorstandzeiten von 18.000 Stunden und mehr zu erreichen, wobei spezifische Katalysatorverbräuche von 0,1 Gew.-% oder weniger erreicht werden. Damit liegen die technischen Vorteile des erfindungsgemäßen Verfahrens außer in der durch den nahezu quantitativen Umsatz bedingten hohen Ausbeute und den ökologischen Vorteilen, die sich aus der Reinheit des hergestellten Produktes ergeben, dem extrem niedrigen Katalysatorverbrauch und den niedrigen Katalysatorkosten.

Die den Reaktor verlassende wäßrige Lösung des Zuckeralkohols kann nach der Entspannung, bei welcher man den überschüssigen Wasserstoff abfangen und nach erfolgter Komprimierung und Ergänzung durch weiteren Wasserstoff erneut zum Einsatz bringen kann, bereits als Zuckeraustauschstoff in flüssiger Form Verwendung finden. Das Wasser dieser Lösung läßt sich aber auf verschiedene Weise, beispielsweise über Sprühtrockner, Trockenwalzen oder durch Gefriertrocknung entfernen. Als günstig hat sich erwiesen, die erhaltene im Regelfall glasklare Lösung des Zuckeralkohols in einem Fallfilmverdampfer oder einem ähnlich arbeitenden Gerät auf einen Zuckeralkoholgehalt von etwa 70 bis 80 Gew.-% aufzukonzentrieren und anschließend nach weiterer Eindampfung in einem Vakuum-Kristallisationsgerät unter Kühlung zur teilweisen oder vollständigen Kristallisation zu bringen. Das Kristallisat läßt sich durch einen nachgeschalteten Mahlprozeß und evtl. Siebung auf eine einheitliche Korngröße bringen. Das erhaltene Produkt ist rieselfähig.

Xylit hat einen Schmelzpunkt von 93 bis 94°C. Beim Sorbit können in Abhängigkeit von den Kristallisationsbedingungen verschiedene kristalline Modifikationen anfallen, von denen die γ-Form mit einem Schmelzpunkt von 101°C die stabilste ist.

In Abhängigkeit von den Kristallisationsumständen kann Lactit entweder als Dihydrat mit einem Schmelzpunkt von 76 bis 78°C oder als Monohydrat mit einem Schmelzpunkt von 121 bis 123°C erhalten werden. Die Löslichkeit beider Hydrate in Wasser ist unterschiedlich; das Monohydrat ist weniger löslich als das Dihydrat. Die Hydrate sind nicht hygroskopisch und zeigen daher technologische Vorteile gegenüber anderen Polyolen. Wasserfreier Lactit kann aus Lösungen in absolutem Ethanol in kristalliner Form gewonnen werden. Er schmilzt bei 146°C und ist nicht hygroskopisch.

Wasserfreier Maltit kann aus Lösungen in absolutem Ethanol nach Animpfen mit Impfkristallen als kristallines Pulver gewonnen werden. Er schmilzt bei 146 bis 148°C und ist hygroskopisch (Helv. Chem. Acta 20, (1937), 86 bis 90). Alle erfindungsgemäß hergestellten Zuckeralkohole haben einen Gehalt an Katalysatorbestandteilen von unter 3 ppm; sie sind in der Regel epimerenfrei.

Als "epimerenfrei" im Sinne der vorliegenden Erfindung gilt ein für die Reinheit der genannten Zuckeralkohole insoweit vernachlässigbarer Gehalt an Epimeren, daß diese Zuckeralkohole die handelsüblichen Spezifikationen, wie sie im Deutschen Arzneimittelbuch (DAB) sowie in der United States Pharmacopeia (USP) und im Food Chemicals Codex (FCC) angegeben sind, ohne weitere Reinigungsgänge erfüllen.

Die Zuckeralkohole werden im erfindungsgemäßen Verfahren in nahezu quantitativer Ausbeute erhalten. Dieses ist von besonderer Bedeutung, da die Entfernung (durch Etherbildung entstandener) höhermolekularer oder (durch Hydrogenolyse entstandener) niedermolekularer störender Verunreinigungen aus dem Reaktionsprodukt durch zusätzliche Reinigungsprozesse, wie Umkristallisation aus Lösungsmitteln normalerweise einen beträchtlichen ökologischen Aufwand hinsichtlich deren Entsorgung erfordert. Die dem Xylit epimeren Zuckeralkohole vom Typ des Lyxits (bzw. Arabinits und Ribits) sind im Reaktionsprodukt höchstens spurenweise (Summe <0,3 Gew.-%) nachweisbar. Der zum Sorbit epimere Mannit ist im Reaktionsprodukt höchstens spurenweise (<0,3 Gew.-%) enthalten. Der zum Lactit diastereomere 4-O-β-D-Galactopyranosyl-D-mannit und der zum Maltit diastereomere 4-O-α-D-Glucopyranosyl-D-mannit sind im jeweiligen Reaktionsprodukt nicht nachweisbar.

Die erfindungsgemäß einzusetzenden sauerstofffreien und trägerfreien Festbettkatalysatoren neigen im Gegensatz zu trägerhaltigen Katalysatoren nicht zum "Ausbluten", d.h. nicht zum Übergang von Katalysatorbestandteilen in ionischer oder kolloidaler Form in die Lösungsphase des Substrats, so daß das Substrat nicht durch Schwermetalle kontaminiert wird, die normalerweise ebenfalls nur mühsam, beispielsweise mit Hilfe von Ionenaustauschern, aus dem Substrat entfernt werden können. Die einzusetzenden Katalysatormetalle können nach ihrem Gebrauch leicht aufgearbeitet und wiederverwendet werden, da die Schwermetalle nicht umständlich von einem Trägermaterial getrennt werden müssen. Bei Polyhydroxylverbindungen war weiterhin die Neigung zu befürchten, daß mit Schwermetallionen komplexe Chelatverbindungen gebildet werden, die nur schwierig aus den Zuckeralkohol-Lösungen entfernt werden können.

Die Süßkraft von Xylit erreicht etwa 80 bis 100 % der Süßkraft von Saccharose.

Wegen seines angenehmen Geschmacks eignet sich Xylit als Zuckeraustauschstoff in der Diabetiker-Diät und als nicht kariogenes Süßungsmittel in Süßwaren und oralen Pharmazeutika. Für Diabetiker-Lebensmittel ist Xylit durch die Diätverordnung (Bundesgesetzblatt 1, 1982, Seite 71) ohne Mengenbegrenzung zugelassen.

Xylit ist besonders gut zur Verarbeitung von Süßwaren, wie Bonbons und Kaugummi, geeignet (Swiss Dent. 1, (7/8) 1980, Seite 25 bis 27). Auch Produkte zur Behandlung des Mund- und Rachenraumes, wie Zahnpasten, rachendesinfizierende Tabletten, Hustenbonbons, werden zunehmend mit Xylit gesüßt (Swiss Dent. III, (7/8) 1982, Seiten 25 bis 30).

Die Süßkraft von Sorbit erreicht etwa 50 bis 60 % der Süßkraft von Saccharose. Zur Erhöhung der Süßkraft kann die wäßrige Lösung mit künstlichen Süßstoffen, beispielsweise Cyclohexylsulfamat oder Asparaginsäure-phenylalanin-methylester, versetzt und durch gemeinsame Vakuumkristallisation in kristalliner Form gewonnen werden. Man kann aber auch die künstlichen Süßstoffe in fester Form mit dem Sorbit-Kristallisat vermischen. Sorbit kann auch in flüssiger oder fester Form mit anderen süß schmeckenden Zuckeralkoholen, beispielsweise Xylit, Maltit, Lactit und anderen, vermischt werden.

Sorbit wird im menschlichen Körper nur in untergeordnetem Maße resorbiert und nur in diesem Anteil abgebaut. Daher ist Sorbit als Zuckerersatzstoff für Diabetiker und als kalorienarmer Süßstoff geeignet. Ferner ist er weniger kariogen als Saccharose oder andere Zucker.

Lactit wird im menschlichen Körper nicht als Kohlehydrat abgebaut und im Dünndarm weder hydrolysiert noch absorbiert. Daher ist Lactit als Zuckerersatzstoff für Diabetiker geeignet. Ferner ist er weniger kariogen als Saccharose.

Die Süßkraft von Lactit erreicht etwa 40 % der Süßkraft von Saccharose. Zur Erhöhung der Süßkraft kann die wäßrige Lösung, wie bei Sorbit mit künstlichen Süßstoffen versetzt und durch gemeinsame Vakuumkristallisation in kristalliner Form gewonnen werden. Man kann aber auch die künstlichen Süßstoffe in fester Form mit dem Lactit-Kristallisat vermischen. Lactit kann außerdem in flüssiger oder fester Form mit anderen süß schmeckenden Zuckeralkoholen, beispielsweise Sorbit, Xylit und anderen, vermischt werden.

Toxische Effekte mit Xylit, Sorbit und Lactit konnten auch in Langzeitstudien nicht festgestellt werden (Ullmanns Encyclopädie der technischen Chemie, Band 24, Weinheim 1983, S. 779), so daß sich vielseitige Anwendungsmöglichkeiten im Lebensmittelbereich, bei der Herstellung von Diabetikerprodukten sowie von zuckerfreien Süßigkeiten und Nahrungsmitteln mit geringem Nährwert ergeben.

Maltit wird im menschlichen Körper durch amylolytische Enzyme nur schwer abgebaut. Daher ist Maltit als Zuckerersatzstoff für kalorienreduzierte Diät und für Diabetiker geeignet (Ullmanns Encyclopädie der technischen Chemie, Band 24, Weinheim 1983, S. 771).

Die Süßkraft von Maltit erreicht die Süßkraft von Saccharose. Maltit kann in flüssiger Form mit anderen süßschmeckenden Zuckeralkoholen, beispielsweise Sorbit, Xylit und anderen, vermischt werden. Besonders der Einsatz in der Getränkeindustrie empfiehlt sich wegen seiner hohen Süßkraft und seiner geringen Neigung zur Kristallisation selbst in hohen Konzentrationen.

Die Prozentangaben der nachfolgenden Beispiele beziehen sich jeweils auf das Gewicht.

### Beispiele

### Beispiel 1

Ein senkrecht stehendes, wärmeisoliertes Hochdruckrohr aus nichtrostendem Stahl von 45 mm Innendurchmesser und 1 m Länge wurde mit 1,4 l eines durch Tablettierung eines Metallpulvers aus einer Ni/Mo-Legierung mit einem Mo-Gehalt von 1,74 % hergestellten Hydrierungskatalysators gefüllt, der bei einer Zylinderhöhe von 5 mm und einem Durchmesser von 5 mm eine Druckfestigkeit von 191 N auf die Zylindermantelfläche und eine innere Oberfläche von 58 m²/g aufwies. Durch dieses Rohr wurden gemeinsam mit der dreifach molaren Menge von unter einem Druck von 300 bar stehendem, hochreinem Wasserstoff stündlich 250 ml einer 40 %igen Lösung von D-Xylose in entionisiertem sauerstofffreiem Trinkwasser mit einem pH-Wert von 7,0 kontinuierlich gepumpt, und zwar von unten nach oben aufsteigend.

Wäßrige Lösung und Wasserstoff wurden vorab gemeinsam durch einen Wärmeaustauscher geführt und so hoch erhitzt, daß sie in das Hochdruckrohr mit einer Temperatur von 70°C eintraten. Das das Hochdruckrohr verlassende Gemisch von wäßriger Lösung und überschüssigem Wasserstoff wurde über einen Kühler in einen Abscheider geführt, von wo der Wasserstoff nach Ersatz der verbrauchten Menge wieder zusammen mit frischer D-Xylose-Lösung in den Vorwärmer und von da erneut in das Hochdruckrohr gepumpt wurde. Die farblose und klare wäßrige Lösung wurde entspannt und in einem Fallfilmverdampfer auf einen Zuckeralkoholgehalt von ca. 75 % aufkonzentriert und anschließend nach weiterer Eindampfung in einem Vakuumkristaller unter Kühlung zur Kristallisation gebracht. Man erhielt ein weißes, leicht hygroskopisches, geruchloses Festprodukt, das zu einem feinkristallinen Pulver verarbeitet wurde. Der gebildete Xylit war ansonsten hochrein und zeigte in der stabilen rhombischen Kristallform einen Schmelzpunkt von 93 bis 94°C. Der Gehalt an nicht hydrierter D-Xylose lag bei ≼0,2 %. Ni- und Mo-Gehalt lagen bei <1 ppm. Der Katalysator war auch nach einer Laufzeit von 2600 Stunden unverändert wirksam.

### Beispiel 2

Durch ein Hochdruckrohr wie in Beispiel 1, aber aus Hochdruckstahl N9, wurde bei einer Temperatur von 65°C und einem Wasserstoffdruck von 150 bar der Wasserstoff im umgekehrten Reaktionsfluß wie in Beispiel 1 der aufsteigenden Lösung von D-Xylose entgegengeführt, wobei stündlich eine gleichgroße Menge einer 40 %igen wäßrigen Lösung von D-Xylose hydriert wurde, die einen pH-Wert von 7,0 aufwies. Der Katalysator war durch Tablettierung von Metallpulver aus einer Ni/Cr-Legierung mit einem Cr-Gehalt von 2,1 %, die zusätzlich mit einem Ti-Anteil von 4,5 % legiert war, hergestellt worden. Die Tabletten hatten bei einer Zylinderhöhe von 5 mm und einem Durchmesser von 5 mm eine Druckfestigkeit von 210 N auf die Zylindermantelfläche und eine innere Oberfläche von 71 m²/g. Nach einer Laufzeit von 1800 Stunden mit unverminderter Wirksamkeit lag der Umsatz der D-Xylose bei ≽99,8 %. Der Gehalt an nicht hydrierter D-Xylose im auskristallisierten Xylit, der einen Reinheitsgrad von ≽99,5 % aufwies, betrug 0,2 %. Ni- und Cr-Gehalt lagen bei 1 ppm.

### Beispiel 3

In einem Hochdruckrohr wie in Beispiel 1 wurde bei einer Temperatur von 65°C und einem Wasserstoffdruck von 300 bar in gleicher Weise wie in Beispiel 1 stündlich eine gleich große Menge einer 40 %igen wäßrigen Lösung von D-Xylose hydriert, die einen pH-Wert von 7,5 aufwies. Der Katalysator war durch Tablettierung einer Ni/Fe/Cr-Legierung gewonnen worden. Die Legierung enthielt einen Fe-Anteil in Ni von 15 % sowie einen Cr-Anteil von 4,5 %. Die Tabletten hatten bei einer Zylinderhöhe von 5 mm und einem Durchmesser von 5 mm eine Druckfestigkeit von 167 N auf die Zylindermantelfläche und eine innere Oberfläche von 68 m²/g. Der in einem Vakuumkristaller unter Animpfung mit Impfkristallen gewonnene kristalline Xylit hatte einen Reinheitsgrad von ≽99,5 %. Der Gehalt an nicht umgesetzter D-Xylose lag bei 0,1 %. Ni-, Fe- und Cr-Gehalt lagen bei <1 ppm. Der Katalysator war nach einer Laufzeit von 1200 Stunden noch unverändert wirksam.

### Beispiel 4

In einem Hochdruckrohr wie in Beispiel 1 wurden bei einer Temperatur von 80°C und einem Wasserstoffdruck von 300 bar in gleicher Weise wie in Beispiel 1 stündlich 150 ml einer 40 %igen wäßrigen Lösung von D-Xylose hydriert, die einen pH-Wert von 6,5 aufwies. Der Katalysator wurde durch Tablettierung einer Co/Mo-Legierung mit einem Mo-Gehalt von 3,8 % gewonnen. Die Tabletten hatten bei einer Zylinderhöhe von 5 mm und einem Durchmesser von 5 mm eine Druckfestigkeit von 185 N auf die Zylindermantelfläche und eine innere Oberfläche von 44 m²/g. Der in einem Vakuumdrehrohr gewonnene Xylit zeigte einen Gehalt an nicht umgesetzter D-Xylose von ≼0,2 %. Der Co-, Mo-Gehalt lag bei <1 ppm. Der Katalysator war nach einer Laufzeit von 1000 Stunden noch unvermindert wirksam.

### Beispiel 5

Ein Hochdruckrohr wie in Beispiel 1 wurde mit 1,4 l eines durch Tablettierung von Metallpulver aus einer Ni/Mo-Legierung mit einem Mo-Gehalt von 1,75 % hergestellten Hydrierungskatalysators gefüllt, der bei einer Zylinderhöhe von 5 mm und einem Durchmesser von 5 mm eine Druckfestigkeit von 191 N auf die Zylindermantelfläche und eine innere Oberfläche von 58 m²/g aufwies. Durch dieses Rohr wurden gemeinsam mit der dreifach molaren Menge von unter einem Druck von 200 bar stehendem hochreinem Wasserstoff stündlich 250 ml einer 40 %igen Lösung von α-D-Glucose in entionisiertem sauerstofffreiem Trinkwasser mit einem pH-Wert von 7,0 kontinuierlich gepumpt, und zwar von unten nach oben aufsteigend. Wäßrige Lösung und Wasserstoff wurden vorher gemeinsam durch einen Wärmeaustauscher geführt und so hoch erhitzt, daß sie in das Hochdruckrohr mit einer Temperatur von 70°C eintraten. Das das Hochdruckrohr verlassende Gemisch von wäßriger Lösung und überschüssigem Wasserstoff wurde über einen Kühler in einen Abscheider geführt, von wo der Wasserstoff nach Ersatz der verbrauchten Menge wieder zusammen mit frischer α-D-Glucose-Lösung in den Vorwärmer und von da erneut in das Hochdruckrohr gepumpt wurde. Die farblose und klare wäßrige Lösung wurde entspannt und in einem Fallfilmverdampfer auf einen Zuckeralkoholgehalt von ca. 70 % aufkonzentriert und anschließend nach weiterer Eindampfung in einem Vakuumkristaller unter Kühlung zur Kristallisation gebracht. Es wurde ein weißes, leicht hygroskopisches, geruchloses Festprodukt erhalten, das zu einem feinkristallinen Pulver vermahlen wurde. Der gebildete Sorbit war ansonsten hochrein und zeigte in der stabilen γ-Form einen Schmelzpunkt von 101°C. Der Gehalt an nicht hydrierter α-D-Glucose lag bei ≼0,1 %. Ni- und Mo-Gehalt lagen bei <1 ppm. Der Katalysator war auch nach einer Laufzeit von 4200 Stunden unverändert wirksam.

### Beispiel 6

Durch ein Hochdruckrohr wie in Beispiel 1 wurde bei einer Temperatur von 75°C und einem Wasserstoffdruck von 300 bar der Wasserstoff im umgekehrten Reaktionsfluß, wie in Beispiel 1 beschrieben, der aufsteigenden Lösung von α-D-Glucose entgegengeführt, wobei stündlich eine gleichgroße Menge 40 %iger wäßriger Lösung von α-D-Glucose wie im Beispiel 1 hydriert wurde, die einen pH-Wert von 7,0 aufwies. Der Katalysator war durch Tablettierung eines Metallpulvers aus einer Ni/Cr-Legierung mit einem Cr-Gehalt von 2,1 %, die mit einem Ti-Anteil von 4,5 % legiert war, hergestellt worden. Die Tabletten hatten bei einer Zylinderhöhe von 5 mm und einem Durchmesser von 5 mm eine Druckfestigkeit von 210 N auf die Zylindermantelfläche und eine innere Oberfläche von 71 m²/g. Nach einer Laufzeit von 2200 Stunden mit unverminderter Wirksamkeit lag der Umsatz der α-D-Glucose bei 99,9 %. Der Gehalt an nicht hydrierter α-D-Glucose im auskristallisierten Sorbit, der einen Reinheitsgrad von ≽99,5 % aufwies, betrug ≼0,1 %. Der Ni-Gehalt lag bei <1 ppm. Der Cr- und Ti-Gehalt lagen bei 1 ppm.

### Beispiel 7

In einem Hochdruckrohr wie in Beispiel 1 wurde bei einer Temperatur von 65°C und einem Wasserstoffdruck von 300 bar in gleicher Weise wie in Beispiel 1 stündlich eine gleichgroße Menge einer 40 %igen wäßrigen Lösung von α-D-Glucose hydriert, die einen pH-Wert von 7,5 aufwies. Der Katalysator war durch Tablettierung einer pulverisierten Ni/Fe/Cr-Legierung gewonnen worden. Die Legierung enthielt einen Fe-Anteil in Ni von 15 % sowie einen Cr-Anteil von 4,5 %. Die Tabletten hatten bei einer Zylinderhöhe von 5 mm und einem Durchmesser von 5 mm eine Druckfestigkeit von 167 N auf die Zylindermantelfläche und eine innere Oberfläche von 68 m²/g. Der in einem Vakuumkristaller gewonnene kristalline Sorbit hatte einen Reinheitsgrad von ≽99,5 %. Der Gehalt an nicht umgesetzter α-D-Glucose lag bei 0,2 %. Ni-, Cr- und Fe-Gehalt lagen jeweils bei <1 ppm. Der Katalysator war nach einer Laufzeit von 1400 Stunden noch unverändert wirksam.

### Beispiel 8

In einem Hochdruckrohr wie in Beispiel 1 wurden bei einer Temperatur von 70°C und einem Wasserstoffdruck von 300 bar in gleicher Weise wie in Beispiel 1 stündlich 150 ml einer 35 %igen wäßrigen Lösung von α-D-Glucose hydriert, die einem pH-Wert von 6,5 aufwies. Der Katalysator war durch Tablettierung von Metallpulver einer Ni/Mo-Legierung mit einem Mo-Gehalt von 1,6 %, die zusätzlich einen Al-Gehalt von 6,1 % aufwies, gewonnen worden. Die Tabletten hatten bei einer Zylinderhöhe von 5 mm und einem Durchmesser von 5 mm eine Druckfestigkeit von 210 N auf die Zylindermantelfläche und eine innere Oberfläche von 71 m²/g. Der in einem Vakuumdrehrohr gewonnene Sorbit zeigte einen Gehalt an nicht umgesetzter α-D-Glucose von ≼0,2 %. Ni-, Al- und Mo-Gehalt lagen bei 1 ppm. Der Katalysator war nach einer Laufzeit von 1300 Stunden noch unvermindert wirksam.

### Beispiel 9

Ein Hochdruckrohr wie in Beispiel 1 wurde mit 1,4 l eines durch Tablettierung von Metallpulver aus einer Ni/Fe/Mo-Legierung mit einem Fe-Anteil von 15 % sowie einem Mo-Anteil von 1,4 % hergestellten Hydrierungskatalysator gefüllt, der bei einer Zylinderhöhe von 3 mm und einem Durchmesser von 3 mm eine Druckfestigkeit von 162 N auf die Zylindermanteloberfläche und eine innere Oberfläche von 68 m²/g aufwies. Durch dieses Rohr wurden gemeinsam mit der dreifach molaren Menge von unter einem Druck von 300 bar stehendem, hochreinem Wasserstoff stündlich 200 ml einer 40 %igen Lösung von 4-O-β-D-Galactopyranosyl-α-D-glucopyranose in entionisiertem sauerstofffreiem Trinkwasser mit einem pH-Wert von 7,0 kontinuierlich gepumpt, und zwar von unten nach oben aufsteigend. Wäßrige Lösung und Wasserstoff wurden vorher gemeinsam über einen Wärmetauscher geführt und so hoch erhitzt, daß sie in das Hochdruckrohr mit einer Temperatur von 70°C eintraten. Das das Hochdruckrohr verlassende Gemisch von wäßriger Lösung und überschüssigem Wasserstoff wurde über einen Kühler in einen Abscheider geführt, von wo der Wasserstoff nach Ersatz der verbrauchten Menge wieder zusammen mit frischer 4-O-β-D-Galactopyranosyl-α-D-glucopyranose in den Vorwärmer und von da erneut in das Hochdruckrohr gepumpt wurde. Die farblose und klare wäßrige Lösung wurde entspannt und in einem Fallfilmverdampfer auf einen Zuckeralkoholgehalt von ca. 80 % aufkonzentriert und anschließend nach weiterer Eindampfung in einem Vakuumkristaller unter Kühlung zur Kristallisation gebracht. In Abhängigkeit von den Kristallisationsumständen und dem Restwassergehalt der eingedampften Lösung ließ sich dabei entweder das Dihydrat mit einem Schmelzpunkt von 76 bis 78°C oder das Monohydrat mit einem Schmelzpunkt von 121 bis 123°C isolieren; der gebildete 4-O-β-D-Galactopyranosyl-D-sorbit war ansonsten rein (Reinheitsgrad ≽99,6 %). Der Gehalt an nicht hydrierter 4-O-β-D-Galactopyranosyl-α-D-glucopyranose lag bei ≼0,2 %. Der Gehalt an Sorbit betrug ≼0,1 %. 4-O-β-D-Galactopyranosyl-D-mannit bzw. Mannit konnten nicht nachgewiesen werden. Ni-Gehalt, Fe-Gehalt und Mo-Gehalt lagen bei 1 ppm. Der Katalysator war auch nach einer Laufzeit von 1700 Stunden unverändert wirksam.

### Beispiel 10

Durch ein Hochdruckrohr, wie in Beispiel 1 verwendet, wurde bei einer Temperatur von 65°C und einem Wasserstoffdruck von 150 bar der Wasserstoff im umgekehrten Reaktionsfluß, wie in Beispiel 1 beschrieben, der aufsteigenden Lösung von 4-O-β-D-Galactopyranosyl-α-D-glucopyranose entgegengeführt, wobei stündlich eine gleichgroße Menge 40 %iger wäßriger Lösung von 4-O-β-D-Galactopyranosyl-α-D-glucopyranose wie in Beispiel 1 hydriert wurde, die einen pH-Wert von 6,5 aufwies. Der Katalysator wurde durch Tablettierung aus Metallpulver einer Ni/Mo/Al-Legierung mit einem Mo-Anteil von 1,62 % und einem Al-Anteil von 6,1 % hergestellt. Die Tabletten hatten bei einer Zylinderhöhe von 5 mm und einem Durchmesser von 5 mm eine Druckfestigkeit von 210 N auf die Zylindermantelfläche und eine innere Oberfläche von 71 m²/g. Nach einer Laufzeit von 1800 Stunden mit unverminderter Wirksamkeit lag der Gehalt an 4-O-β-D-Galactopyranosyl-D-sorbit des in einem Rotationsverdampfer zur Trockne eingedampften Reaktionsgemisches bei 99,4 %. Der Gehalt an nicht hydrierter 4-O-β-D-Galactopyranosyl-α-D-glucopyranose betrug ≼0,2 %. Der Gehalt an Sorbit lag bei 0,1 %. 4-O-β-D-Galactopyranosyl-D-mannit bzw. Mannit konnten nicht nachgewiesen werden. Ni-, Mo- und Al-Gehalt lagen bei <3 ppm.

### Beispiel 11

In einem Hochdruckrohr, wie in Beispiel 1 verwendet, wurde bei einer Temperatur von 70°C und einem Wasserstoffdruck von 300 bar in gleicher Weise wie in Beispiel 1 stündlich eine gleichgroße Menge einer 40 %igen wäßrigen Lösung von 4-O-β-D-Galactopyranosyl-α-D-glucopyranose hydriert, die einen pH-Wert von 7,5 aufwies. Der Katalysator wurde durch Tablettierung einer pulverisierten Ni/Cr/Mn-Legierung gewonnen. Die Legierung enthielt einen Cr-Anteil von 3,8 % und einen Mn-Anteil von 2,4 %. Die Tabletten hatten bei einer Zylinderhöhe von 5 mm und einem Durchmesser von 5 mm eine Druckfestigkeit von 195 N auf die Zylindermantelfläche und eine innere Oberfläche von 74 m²/g. Der in einem Vakuumkristaller gewonnene 4-O-β-D-Galactopyranosyl-α-D-sorbit hatte einen Reinheitsgrad von 99,3 %. Der Gehalt an nicht umgesetzter 4-O-β-D-Galactopyranosyl-α-D-glucopyranose lag bei 0,1 %. Der Sorbitgehalt betrug 0,1 %. 4-O-β-D-Galactopyranosyl-D-mannit bzw. Mannit konnten nicht nachgewiesen werden. Ni-, Cr-, Mn-Gehalt lagen bei <2 ppm. Der Katalysator war nach einer Laufzeit von 1994 Stunden noch unverändert wirksam.

### Beispiel 12

In einem Hochdruckrohr, wie in Beispiel 1 verwendet, wurde bei einer Temperatur von 65°C und einem Wasserstoffdruck von 200 bar in gleicher Weise wie in Beispiel 1 eine ebenso große Menge einer 30 %igen wäßrigen Lösung von 4-O-β-D-Galactopyranosyl-α-D-glucopyranose hydriert, die einen pH-Wert von 6,5 aufwies. Der Katalysator enthielt einen Mo-Anteil von 1,61 % sowie einen Al-Anteil von 6,1 %. Die Tabletten hatten bei einer Zylinderhöhe von 5 mm und einem Durchmesser von 5 mm eine Druckfestigkeit von 210 N auf die Zylindermantelfläche und eine innere Oberfläche von 71 m²/g. Der in einem Vakuumdrehrohr gewonnene 4-O-β-D-Galactopyranosyl-α-D-sorbit hatte einen Reinheitsgrad von 99,4 %. Der Gehalt an nicht umgesetzter 4-O-β-D-Galactopyranosyl-α-D-glucopyranose lag bei 0,2 %. Der Sorbitgehalt betrug 0,2 %. 4-O-β-D-Galactopyranosyl-D-mannit konnte nicht nachgewiesen werden. Der Katalysator war nach einer Laufzeit von 1900 Stunden noch unvermindert wirksam.

### Beispiel 13

Ein Hochdruckrohr wie in Beispiel 1 wurde mit 1,4 l eines durch Tablettierung von Metallpulver aus einer Legierung von Ni/Cr/Ti mit einem Cr-Gehalt von 2,1 % und einem Ti-Gehalt von 4,5 % hergestellten Hydrierungskatalysators gefüllt, der bei einer Zylinderhöhe von 5 mm und einem Durchmesser von 5 mm eine Druckfestigkeit von 210 N auf die Zylindermantelfläche und eine innere Oberfläche von 71 m²/g aufwies. Durch dieses Rohr wurden gemeinsam mit der fünffach molaren Menge von unter einem Druck von 300 bar stehendem, hochreinem Wasserstoff stündlich 250 ml einer 40 %igen wäßrigen Lösung von 4-O-β-D-Galactopyranosyl-α-D-glucopyranose in entionisiertem sauerstoffreiem Trinkwasser mit einem pH-Wert von 7,0 kontinuierlich gepumpt, und zwar von unten nach oben aufsteigend. Wäßrige Lösung und Wasserstoff wurden vorab gemeinsam durch einen Wärmetauscher geführt und so hoch erhitzt, daß sie in das Hochdruckrohr mit einer Temperatur von 60°C eintraten. Das das Hochdruckrohr verlassende Gemisch von wäßriger Lösung und überschüssigem Wasserstoff wurde über einen Kühler in einen Abscheider geführt, von wo der Wasserstoff nach Ersatz der verbrauchten Menge wieder zusammen mit frischer 4-O-β-D-Galactopyranosyl-α-D-glucopyranose in den Vorwärmer und von da erneut in das Hochdruckrohr gepumpt wurde. Die farblose und klare wäßrige Lösung wurde entspannt und in einem Fallfilmverdampfer auf einen Zuckeralkoholgehalt von ca. 80 % aufkonzentriert und anschließend nach weiterer Eindampfung in einem Vakuumkristaller unter Kühlung und gegebenenfalls Hinzufügung von Impfkristallen zur Kristallisation gebracht. Der kristalline 4-O-β-D-Galactopyranosyl-D-sorbit zeigte einen Reinheitsgehalt von ≽99,3 %. Der Gehalt an nicht hydrierter 4-O-β-D-Galactopyranosyl-α-D-glucopyranose lag bei ≤0,2 %. Der Gehalt an Sorbit betrug ≼0,3 %. Mannit konnte nicht nachgewiesen werden. Der Katalysator war auch nach einer Laufzeit von 2800 Stunden unverändert wirksam.

### Beispiel 14

Durch ein Hochdruckrohr, wie in Beispiel 1 verwendet, wurde bei einer Temperatur von 70°C und einem Wasserstoffdruck von 200 bar der Wasserstoff in umgekehrten Reaktionsfluß, wie in Beispiel 1 beschrieben, der aufsteigenden Lösung von 4-O-β-D-Galactopyranosyl-α-D-glucopyranose entgegengeführt, wobei stündlich eine gleich große Menge 40 %iger wäßriger Lösung von 4-O-β-D-Galactopyranosyl-α-D-glucopyranose wie in Beispiel 1 hydriert wurde, die einen pH-Wert von 6,5 aufwies. Der Katalysator wurde durch Tablettierung von Ni/Mo/Al-Pulver mit einem Mo-Gehalt von 1,6 % und einem Al-Anteil von 6,1 % hergestellt. Die Tabletten hatten bei einer Zylinderhöhe von 5 mm und einem Durchmesser von 5 mm eine Druckfestigkeit von 210 N auf die Zylindermantelfläche und eine innere Oberfläche von 71 m²/g. Nach einer Laufzeit von 1900 Stunden mit unverminderter Wirksamkeit lag der Gehalt an 4-O-β-D-Galactopyranosyl-D-sorbit des in einem Rotationsverdampfer zur Trockne eingedampften Reaktionsgemisches bei 99,2 %. Der Gehalt an nicht hydrierter 4-O-β-D-Galactopyranosyl-α-D-glucopyranose betrug ≼0,2 %. Der Gehalt an Sorbit lag bei 0,3 %.

### Beispiel 15

In einem Hochdruckrohr, wie in Beispiel 1 verwendet, wurde bei einer Temperatur von 60°C und einem Wasserstoffdruck von 300 bar in gleicher Weise wie in Beispiel 1 stündlich eine gleich große Menge einer 40 %igen wäßrigen Lösung von 4-O-β-D-Galactopyranosyl-α-D-glucopyranose hydriert, die einen pH-Wert von 7,5 aufwies. Der Katalysator wurde durch Tablettierung einer pulverisierten Ni/Fe/Cr-Legierung mit einem Fe-Anteil von 15 % und einem Cr-Gehalt von 4,5 % hergestellt. Die Tabletten hatten bei einer Zylinderhöhe von 5 mm und einem Durchmesser von 5 mm eine Druckfestigkeit von 167 N auf die Zylindermantelfläche und eine innere Oberfläche von 68 m²/g. Der in einem Vakuumverdampfer gewonnene 4-O-β-D-Galactopyranosyl-α-D-sorbit hatte einen Reinheitsgrad von 99,1 %. Der Gehalt an nicht umgesetzter 4-O-β-D-Galactopyranosyl-α-D-glucopyranose lag bei 0,2 %. Der Sorbitgehalt betrug 0,3 %. Ni-, Fe- und Cr-Gehalt lagen bei <3 ppm. Der Katalysator war nach einer Laufzeit von 1200 Stunden noch unverändert wirksam.

### Beispiel 16

In einem Hochdruckrohr, wie in Beispiel 1 verwendet, wurde bei einer Temperatur von 65°C und einem Wasserstoffdruck von 200 bar in gleicher Weise wie in Beispiel 1 eine ebenso große Menge einer 30 %igen wäßrigen Lösung von 4-O-β-D-Galactopyranosyl-α-D-glucopyranose hydriert, die einen pH-Wert von 6,5 aufwies. Der Katalysator wurde durch Tablettierung eines Metallpulvers aus einer Ni/Mo/Al-Legierung mit einem Mo-Gehalt von 0,55 % und einem Al-Gehalt von 6,5 % gewonnen. Die Tabletten hatten bei einer Zylinderhöhe von 5 mm und einem Durchmesser von 5 mm eine Druckfestigkeit von 197 N auf die Zylindermantelfläche und eine innere Oberfläche von 64 m²/g. Der in einem Vakuumdrehrohr gewonnene 4-O-β-D-Galactopyranosyl-α-D-sorbit -α-D-sorbit hatte einen Reinheitsgrad von 99,3 %. Der Gehalt an nicht umgesetzter 4-O-ß-D-Galactopyranosyl-α-D-glucopyranose lag bei 0,1 %. Der Sorbitgehalt betrug 0,3 %. Der Katalysator war nach einer Laufzeit von 3600 Stunden unvermindert wirksam.

## Patentansprüche

1. Verfahren zur Herstellung von Zuckeralkoholen aus der Gruppe von Xylit, Sorbit, 4-O-β-D-Galactopyranosyl-D-sorbit und 4-O-α-D-Glucopyranosyl-D-sorbit durch katalytische Hydrierung der korrespondierenden Zucker D-Xylose, α-D-Glucose, 4-O-β-D-Galactopyranosyl-α-D-glucopyranose bzw. 4-O-α-D-Glucopyranosyl-α-D-glucopyranose in wäßriger Lösung mit Wasserstoff unter erhöhtem Druck und bei erhöhter Temperatur, dadurch gekennzeichnet, daß man die Hydrierung kontinuierlich bei einem Wasserstoffdruck von 100 bis 400 bar und Temperaturen von 40 bis 80°C im Festbettverfahren in einer Reaktionszone über als Hydrierungskatalysatoren dienenden trägerfreien Formkörpern mit einer Druckfestigkeit von 20 bis 250 N und einer inneren Oberfläche von 10 bis 80 m²/g von (i) einem oder mehreren Elementen der Eisenuntergruppe der VIII. Nebengruppe des Periodensystems durchführt, die zusätzlich mit (ii) einem oder mehreren aktivierend wirkenden Element der VI. Nebengruppe im Mengen von mindestens 0,1 bis höchstens 20 Gew.-% bezogen auf den trägerfreien Formkörper legiert sind.

2. Verfahren nach Anspruch 1, dadurch gekennzeichnet, daß es sich bei den Formkörpern um solche aus verpreßten Metallpulvern handelt.

3. Verfahren nach Anspruch 1, dadurch gekennzeichnet, daß die Formkörper, makroskopisch betrachtet, eine glatte Oberfläche aufweisen.

4. Verfahren nach Anspruch 1, dadurch gekennzeichnet, daß die Formkörper tabletten- oder kugelförmig sind und Durchmesser von 3 bis 7 mm besitzen.

5. Verfahren nach Anspruch 1, dadurch gekennzeichnet, daß man die Hydrierung der Zucker in 15 bis 45 %iger wäßriger Lösung bei einem pH-Wert von 3,5 bis 10,5 durchführt.

## Claims

1. Process for the preparation of sugar alcohols selected from the group consisting of xylitol, sorbitol, 4-O-β-D-galactopyranosyl-D-sorbitol and 4-O-α-D-glucopyranosyl-D-sorbitol by catalytic hydrogenation of the corresponding sugars D-xylose, α-D-glucose, 4-O-β-D-galactopyranosyl-α-D-glucopyranose and 4-O-α-D-glucopyranosyl-α-D-glucopyranose, respectively, in aqueous solution with hydrogen at elevated pressure and elevated temperature, characterized in that the hydrogenation is carried out continuously at a hydrogen pressure of 100 to 400 bar, and temperatures of 40 to 80°C, in the fixed-bed process in a reaction zone over support-free shaped bodies serving as hydrogenation catalysts and having a compressive strength of 20 to 250 N, and an internal surface area of 10 to 80 m²/g of (i) one or more elements of the iron subgroup of subgroup VIII of the Periodic Table of the Elements which are additionally alloyed with (ii) one or more elements of subgroup VI having activating activity in amounts of at least 0.1 to at most 20 % by weight, based on the support-free shaped body.

2. Process according to Claim 1, characterized in that the shaped bodies are those composed of pressed metal powders.

3. Process according to Claim 1, characterized in that the shaped bodies viewed macroscopically have a smooth surface.

4. Process according to Claim 1, characterized in that the shaped bodies are tablet-shaped or spherical and have diameters from 3 to 7 mm.

5. Process according to Claim 1, characterized in that the hydrogenation of the sugars is carried out in 15 to 45 % aqueous solution at a pH of 3.5 to 10.5.

## Revendications

1. Procédé de production d'alcools de sucre du groupe du xylitol, du sorbitol, du 4-O-β-D-galactopyranosyl-D-sorbitol et du 4-O-α-D-glucopyranosyl-D-sorbitol par hydrogénation catalytique des sucres correspondants D-xylose, α-D-glucose, 4-O-β-D-galactopyranosyl-α-D-glucopyranose et 4-O-α-D-glucopyranosyl-α-D-glucopyranose en solution aqueuse avec de l'hydrogène sous pression et à température augmentée, caractérisé en ce que l'on conduit l'hydrogénation en continu à une pression d'hydrogène de 100 à 400 bar et à des températures de 40 à 80°C, dans le procédé en lit fixe dans une zone réactionnelle sur des corps façonnés sans support qui servent de catalyseurs d'hydrogénation ayant une résistance à l'écrasement de 20 à 250 N, et une surface interne de 10 à 80 m²/g de (i) un ou plusieurs éléments du sous-groupe du fer du VIII^{ème} groupe secondaire du système périodique, qui sont en outre alliés avec (ii) un ou plusieurs éléments à effet activant du VI^{ème} groupe secondaire en des quantités d'au moins 0,1 à au plus 20 % en masse par rapport aux corps façonnés sans support.

2. Procédé selon la revendication 1, caractérisé en ce que les corps façonnés sont des corps façonnés constitués par des poudres métalliques comprimées.

3. Procédé selon la revendication 1, caractérisé en ce que, quand ils sont observés macroscopiquement, les corps façonnés ont une surface lisse.

4. Procédé selon la revendication 1, caractérisé en ce que les corps façonnés sont en forme de comprimés ou de sphères et possèdent un diamètre de 3 à 7 mm.

5. Procédé selon la revendication 1, caractérisé en ce que l'on réalise l'hydrogénation des sucres dans une solution aqueuse à 15 à 45 % à un pH de 3,5 à 10,5.
